# EUROPEAN PATENT APPLICATION

(11) **EP 4 685 811 A1**
(43) Date of publication of application: **28.01.2026**
(21) Application number: 24190512.4
(22) Date of filing: 24.07.2024
(51) Int. Cl.: G16H 40/67, A61B 5/00, A61B 5/11

(54) **SYSTEM FOR PROVIDING A THERAPEUTIC AND/OR DIAGNOSTIC FUNCTION ON A PATIENT**

(71) Applicant: BIOTRONIK SE & Co. KG, 12359 Berlin (DE)
(72) Inventor: FRYER, Alan, Portland, 97202 (US)
(74) Representative: Biotronik Corporate Services SE

(57) **Abstract**

A system for providing a therapeutic and/or diagnostic function on a patient comprises an implantable medical device having a first implant transceiver circuitry, a second implant transceiver circuitry , an implant processing circuitry and an implant motion sensor. The system furthermore comprises an external device having a first device transceiver circuitry, a device processing circuitry and a device motion sensor. The implant processing circuitry is configured to obtain, using the implant motion sensor and based on a communication established via the first implant transceiver circuitry and the first device transceiver circuitry, a first motion signal and to derive, based on the first motion signal, a first authentication token. The device processing circuitry is configured to obtain, using the device motion sensor and based on said communication established via the first implant transceiver circuitry and the first device transceiver circuitry, a second motion signal and to derive, based on the second motion signal, a second authentication token.

## Description

The invention relates to a system for providing a therapeutic and/or diagnostic function on a patient and to a method for operating a system for providing a therapeutic and/or diagnostic function on a patient.

A system of this kind generally comprises an implantable medical device configured for implantation in the patient and having some kind of implant transceiver circuitry, and an implant processing circuitry. A system of this kind furthermore comprises an external device having some kind of device transceiver circuitry and a device processing circuitry. The implantable medical device and the external device, via their transceiver circuitries, are configured to establish a communication with one another in order to exchange information, for example to transmit programming information from the external device to the implantable medical device or to transmit operational data, such as measurement data obtained during operation, from the implantable medical device to the external device.

The implantable medical device may for example be a cardiac stimulation device, such as a cardiac pacemaker device or a cardiac defibrillator device. For example, the implantable medical device may be an IPG, ICD or CRT (CRT-D or CRT-P) device. In other embodiments, the implantable medical device may be a monitoring device for monitoring a medical function. In yet other embodiments, the implantable medical device may be a neuro-stimulation device.

The external device may for example be a wearable device configured to be worn by the patient or an external communication device such as a smart phone or a tablet computer or a programming device or the like.

Within systems of the type concerned herein a secure communication in between the implantable medical device and one or multiple external devices is of utmost importance. A corruption or alternation of a communication between the implantable medical device and the external device or a further device may present an immediate and severe risk for the patient.

For this reason, for establishing a communication in between the implantable medical device and the external device or a further device some kind of authentication scheme generally is applied, and a data exchange is protected by cryptographic techniques. For example, the implantable medical device authenticates the external device or a further device for establishing a communication, upon which a role-based security determined on the type of the external device is applied. This generally requires a shared secret between the two devices or the ability to generate a shared secret in a secure manner.

For example, known approaches to establish a secure communication between an implantable medical device and an external device include the use of pre-shared keys, which are for example stored on the implantable medical device during manufacturing. Other approaches include the use of a pin programmed into the implantable medical device by a clinician program during the implantation procedure of the implantable medical device. In addition, public/private key solutions may be employed for encrypting a data transfer and/or secure shared key derivation.

At least some of the concepts for establishing a secure communication come with the inherent drawback that keys must be exchanged beforehand and rely on a secrecy of shared keys.

US 9,185,087 B2 discloses devices, systems, and techniques for generating an encryption key using detected motion from a device. In one example, a method may include receiving movement information indicative of motion detected by a first device during a period of time in which the first device and a second device were bumped together, determining a set of values that represent at least one characteristic of the movement information, and generating, based on the set of values, an encryption key for at least one of encrypting and decrypting data communicated between the first device and the second device.

US 10,682,517 B2 discloses techniques for facilitating authorized telemetry with an implantable device. In one embodiment, for example, a method includes comparing, by a first device having a processor, first electronic information with second electronic information. The first electronic information is indicative of a first motion of a second device external to a body in which the implantable device is located, and the second electronic information is indicative of a second motion of the implantable device. The method also includes determining whether a defined level of correlation exists between the first electronic information and the second electronic information, and initiating a telemetry session between the second device and the implantable device based on a determination that the defined level of correlation exists between the first electronic information and the second electronic information.

It is an object of the invention to provide a system for providing a therapeutic and/or diagnostic function on a patient and a method for operating such a system which allow for a communication between the implantable medical device and the external device or a further device with an improved security.

In one aspect, a system from for providing a therapeutic and/or diagnostic function on a patient comprises: an implantable medical device configured for implantation in the patient and having a first implant transceiver circuitry, a second implant transceiver circuitry, an implant processing circuitry and an implant motion sensor; an external device having a first device transceiver circuitry, a device processing circuitry and a device motion sensor; wherein the implant processing circuitry is configured to obtain, using the implant motion sensor and based on a communication established via the first implant transceiver circuitry and the first device transceiver circuitry, a first motion signal and to derive, based on the first motion signal, a first authentication token; wherein the device processing circuitry is configured to obtain, using the device motion sensor and based on said communication established via the first implant transceiver circuitry and the first device transceiver circuitry, a second motion signal and to derive, based on the second motion signal, a second authentication token; wherein the implant processing circuitry is configured to authenticate a communication via the second implant transceiver circuitry based on the first authentication token and the second authentication token.

The system comprises an implantable medical device configured for implantation in a patient and an external device. The system furthermore may comprise a further device configured to operate externally to the patient.

The implantable medical device comprises a first implant transceiver circuitry and a second implant transceiver circuitry. The first implant transceiver circuitry and the second implant transceiver circuitry allow to establish communication connections generally according to different communication techniques, such as an inductive coupling technique and a wireless RF communication technique in an RF frequency band.

The external device comprises a first device transceiver circuitry. The first device transceiver circuitry and the first implant transceiver circuitry are configured to establish a communication with one another according to a first communication technique, such as an inductive coupling technique involving an inductive coupling of coil antennas of the first implant transceiver circuitry and the first device transceiver circuitry.

The implantable medical device comprises an implant motion sensor, and the external device comprises a device motion sensor. The implant motion sensor and the device motion sensor each are configured to sense a motion signal. Based on a first motion signal obtained using the implant motion sensor the implant processing circuitry determines a first authentication token, and based on a second motion signal obtained using the device motion sensor the device processing circuitry determines a second authentication token. Based on the first authentication token and the second authentication token, then, an authenticated communication between the implantable medical device and the external device or between the implantable medical device and a further device may be established employing the second implant transceiver circuitry of the implantable medical device.

Within the system a communication between the implantable medical device and the external device or a further device may be improved in terms of security in that authentication tokens are derived based on motion signals. In particular, the implantable medical device in an implanted state rests within the patient such that the implant motion sensor of the implantable medical device will generally pick up a physical body motion of the patient, relating for example to a change in posture or to a breathing motion. In addition, using the external device in close body contact to the patient, the device motion sensor of the external device may be used to also detect body motion, such that the first motion signal obtained using the implant motion sensor and the second motion signal obtained using the device motion sensor are at least to some extent correlated and/or to share the same characteristics. If the implant processing circuitry and the device processing circuitry derive, based on the motion signals obtained using the implant motion sensor and the device motion sensor, authentication tokens according to a common scheme, the authentication tokens may be used to authenticate a communication in between the implantable medical device and the external device or a further device.

Generally, a communication to exchange data between the implantable medical device and the external device or a further device shall be established using the second implant transceiver circuitry according to a second communication technique, for example a wireless RF communication technique. The communication by means of the second communication technique, for example an RF communication technique, shall be authenticated according to the first authentication token and the second authentication token.

For generating the authentication tokens, the implantable medical device and the external device communicate using the first implant transceiver circuitry and the first device transceiver circuitry according to the first communication technique of the first implant transceiver circuitry and the first device transceiver circuitry in order to align the generation of the first authentication token at the implantable medical device and the second authentication token at the external device. By means of the first implant transceiver circuitry and the first device transceiver circuitry a communication in between the implantable medical device and the external device for generating the first authentication token and the second authentication token is established, and based on the communication via the first implant transceiver circuitry and the first device transceiver circuitry the implant processing circuitry obtains the first motion signal using the implant motion sensor and the device processing circuitry obtains the second motion signal using the device motion sensor.

In one embodiment, the device processing circuitry is configured, using the communication established via the first implant transceiver circuitry and the first device transceiver circuitry, to transmit information to the implant processing circuitry relating to a time range for obtaining the first motion signal and the second motion signal.

For example, the device processing circuitry is configured, using the communication established via the first implant transceiver circuitry and the first device transceiver circuitry, to transmit a trigger signal to the implant processing circuitry to start the time range for obtaining the first motion signal and the second motion signal.

In order to obtain the first motion signal at the implantable medical device and the second motion signal at the external device, the time ranges for sensing the motion signals need to be aligned, such that the motion signals relate to the same time range and a physical body motion of the patient occurring in that time range. The alignment of the time range herein takes place using a communication by means of the first implant transceiver circuitry and the first device transceiver circuitry, for example in that the external device triggers a measurement by communicating a trigger signal to the implantable medical device. The measurement of the motion signals at the implantable medical device and at the external device hence is synchronized by means of a communication in between the implantable medical device and the external device using the first implant transceiver circuitry and the first device transceiver circuitry.

The second implant transceiver circuitry, in one embodiment, is configured to establish a communication using a wireless RF communication technique. For example, using the second implant transceiver circuitry an RF communication according to MICS, BLE, or a telemetry scheme may be established.

In one embodiment, the external device comprises a second device transceiver circuitry. The second implant transceiver circuitry and the second device transceiver circuitry make use of the same (second) communication technique, in particular an RF communication technique, such as MICS, BLE or a telemetry scheme.

In one embodiment, the second implant transceiver circuitry and the second device transceiver circuitry are configured to establish an authenticated communication based on the first authentication token and the second authentication token. Once the first authentication token is obtained at the implant and the second authentication token is obtained at the external device, a communication using the second implant transceiver circuitry and the second device transceiver circuitry may be established in an authenticated, secure fashion based on the first authentication token and the second authentication token. In particular, the implantable medical device may authenticate the external device based on the first authentication token and the second authentication token, which should match with one another as they have been generated based on motion signals relating to a common body motion of the patient.

If the authentication based on the authentication tokens is successful, in particular if the first authentication token and the second authentication token match, a data exchange via an authenticated communication session may take place using the second implant transceiver circuitry and the second device transceiver circuitry.

In one embodiment, the external device is configured to transmit information relating to the second authentication token to a further device. The second authentication token hence is generated at the external device, wherein subsequently information relating to the second authentication token, for example the second authentication token itself, is communicated from the external device to the further device.

In one embodiment, the further device comprises a further transceiver circuitry, wherein the second implant transceiver circuitry and the further transceiver circuitry are configured to establish an authenticated communication based on the first authentication token and the second authentication token. The second authentication token hence is generated at the external device, whereupon information relating to the second authentication token, in particular the second authentication token itself, is communicated to the further device, which then may use this information to establish an authenticated communication with the implantable medical device.

The external device hence serves as an authentication means in order to generate, in an aligned session with the implantable medical device, an authentication token, which subsequently may be used by another device in order to establish an authenticated communication with the implantable medical device. Based on the first implant transceiver circuitry the generation of the authentication token at the implantable medical device respectively the external device is aligned, and subsequently a data exchange via an authenticated connection takes place between the implantable medical device and the further device.

The authenticated communication is established based on the first authentication token and the second authentication token, wherein in addition a private key/public key encryption may be used. For example, the first authentication token and the second authentication token may be exchanged between the implantable medical device and the external device respectively the further device using a private key/public key encryption. The authentication tokens main purpose is to authorize the communication, but the session tokens could also be used as an input to generating unique session keys for the subsequent communication exchange. Thus, the authentication tokens may support a generation of a session key in order to allow for a secure, authenticated communication between the implantable medical device and the external device respectively the further device.

In one embodiment, the implant processing circuitry is configured to determine a first characteristic value based on the first motion signal and to derive the first authentication token based on the first characteristic value. The device processing circuitry is configured to determine a second characteristic value based on the second motion signal and to derive the second authentication token based on the second characteristic value.

For example, the implant processing circuitry is configured to determine the first characteristic value based on a set of first characteristic quantities derived from the first motion signal, and the device processing circuitry is configured to determine the second characteristic value based on a set of second characteristic quantities derived from the second motion signal.

The respective characteristic value generally is derived from the respective motion signal. The characteristic value herein is determined based on an analysis of the motion signal and by determining characteristic quantities, relating for example to maxima and minima of the motion signal, to times of occurrence of maxima and minima, to a relative change between maxima and/or minima, to an area under a portion of a curve of the motion signal and the like. Generally, the characteristic values may be determined in any way based on a processing of the motion signals, wherein the characteristic values beneficially are determined such that they robustly are alike, provided that there is a sufficient correlation between the first motion signal obtained at the implantable medical device and the second motion signal at the external device.

The first characteristic value and the second characteristic value for example are expressed by a number of bits or a number of digits, wherein the first characteristic value and the second characteristic value for example may be small in size, for example corresponding to a number having a bit length between 2 bits to 20 bits, in particular between 10 bits to 16 bits (if it used solely for authentication). Approximately 9 bits would be equivalent or better than a standard 4 digit pin numbers and 14 bits would be greater than a 6 digit pin. The entropy required for an authentication is considerably less than that required for encryption as the goal is to protect only a time limited window initiated on application of the first communication signal. Further, the design can actively reject communication in cases of invalid communication attempts to prevent brute force attacks. By chaining characteristics higher bit lengths may still be used.

In one embodiment, the implant processing circuitry is configured to derive the first authentication token from the first characteristic value using a predefined randomizing function.

The device processing circuitry is configured to derive the second authentication token from the second characteristic value using the same predefined randomizing function. The first characteristic value and the second characteristic value in particular may be input to the same pre-defined randomizing function, implemented beneficially by software in the implant processing circuitry and the device processing circuitry, the output of the randomizing function being the first authentication token at the implantable medical device and the second authentication token at the external device. The first authentication token and the second authentication token beneficially each comprise a much larger number of bits, for example 32 bit, 64 bit or 128 bit.

Additional randomizing information, such as information relating to a time of day or the like, may be input into the randomizing function at the implantable medical device and the external device in order to provide for an additional randomization for generating the first authentication token and the second authentication token.

In one embodiment, the external device is a wearable device configured to be worn by the patient. As the wearable external device is worn by the patient, the external device is enabled to sense a body motion correlated to the body motion sensed at the implantable medical device. If the implantable medical device for example is a cardiac device, such as a cardiac pacemaker or defibrillator device, the external device may for example be designed to be worn on the patient's chest, such that it can be assumed that a body motion at the location of the implantable medical device will substantially be correlated with a body motion at the external device.

In other embodiments, the external device may be a communication device such as a smart phone device or a tablet computer or a clinician programming device. For sensing a motion signal using the external device, a user may for example be prompted to place the external device at a defined position on the body of the patient, for example on the chest, such that a body motion may be sensed by the external device which is correlated to a body motion as sensed by the implantable medical device.

In another aspect, a method for operating a system for providing a therapeutic and/or diagnostic function on a patient comprises: providing an implantable medical device configured for implantation in the patient and having a first implant transceiver circuitry, a second implant transceiver circuitry, an implant processing circuitry and an implant motion sensor; providing an external device having a first device transceiver circuitry, a device processing circuitry and a device motion sensor; obtaining, by the implant processing circuitry, a first motion signal using the implant motion sensor and based on a communication established via the first implant transceiver circuitry and the device transceiver circuitry, and deriving, based on the first motion signal, a first authentication token; obtaining, by the device processing circuitry, a second motion signal using the device motion sensor and based on said communication established via the first implant transceiver circuitry and the first device transceiver circuitry, and deriving, based on the second motion signal, a second authentication token; authenticating, by the implant processing circuitry, a communication via the second implant transceiver circuitry based on the first authentication token and the second authentication token.

The advantages and advantageous embodiments as described above for the system equally apply also to the method, such that it shall be referred to the above.

The generation of the first authentication token at the implantable medical device and the second authentication token at the external device may for example be triggered by the external device. For example, the establishing of a communication may be triggered by a user at the external device or at a further device, upon which the implantable medical device and the external device establish a communication using the first implant transceiver circuitry and the first device transceiver circuitry in order to synchronize a sensing of motion signals at the implantable medical device and the external device for generating the first authentication token and the second authentication token. The generation of the authentication tokens for establishing a secure communication hence is substantially at the control of a user, e.g. the patient.

In one embodiment, the patient may be caused to perform a defined motion in a specified time range for allowing the implant processing circuitry to obtain the first motion signal and the device processing circuitry to obtain the second motion signal. For example, prompted by the external device the patient may be caused to hold the external device in a defined position, for example close to the chest of the patient, upon which the patient shall conduct a specified motion, such as a specified physical body motion by for example moving the upper body of the patient in a specified manner. The physical motion is sensed at the implantable medical device and at the external device, such that correlated motion signals are obtained at the implantable medical device and the external device, which are used to generate authentication tokens which subsequently may be used to authenticate a communication in between the implantable medical device and the external device or a further device.

The various features and advantages of the present invention may be more readily under-stood with reference to the following detailed description and the embodiments shown in the drawings. Herein,
- Fig. 1: shows a schematic view of a system comprising an implantable medical device and an external device;
- Fig. 2: shows a schematic drawing of an implantable medical device and an external device;
- Fig. 3: shows a schematic drawing of an implantable medical device and an external device, illustrating a scheme for generating authentication tokens based on motion signals sensed by the implantable medical device and the external device;
- Fig. 4: shows a scheme for generating authentication tokens based on a randomizing function;
- Fig. 5: shows a schematic drawing of a system comprising an implantable medical device, an external device and a further device; and
- Fig. 6: shows a schematic drawing of an implantable medical device, an external device and a further device for establishing a communication.

Subsequently, embodiments of the invention shall be described in detail with reference to the drawings. In the drawings, like reference numerals designate like structural elements.

It is to be noted that the embodiments are not limiting for the invention, but merely represent illustrative examples.

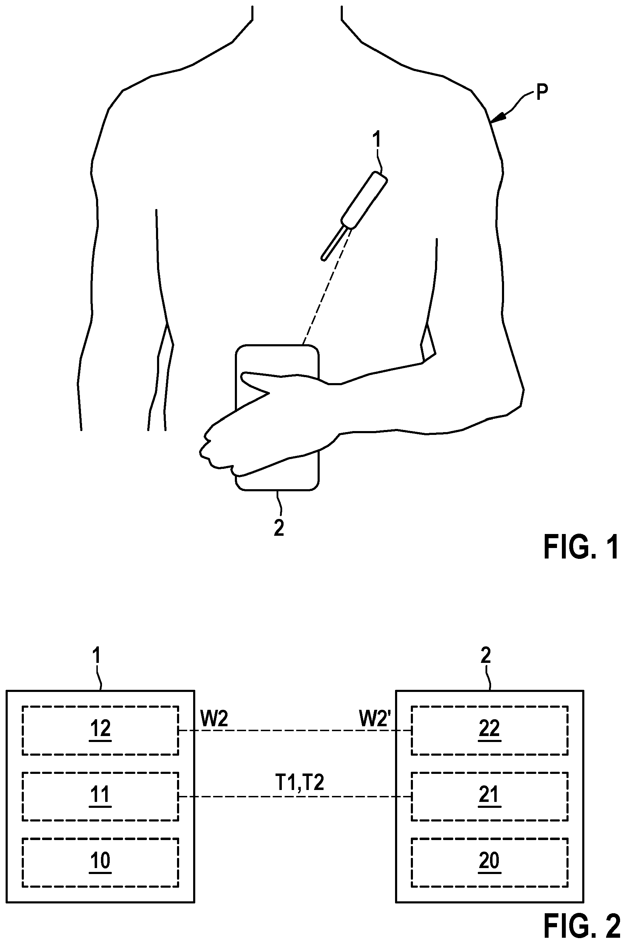

Referring now to Fig. 1, a system for performing a therapeutic and/or diagnostic function on a patient P comprises an implantable medical device 1 configured for implantation in the patient P and an external device 2 configured for operation externally to the patient P.

The implantable medical device 1 may for example be a cardiac stimulation device, such as a cardiac pacemaker device or a cardiac defibrillator device. For example, the implantable medical device 1 is an IPG, ICD or CRT device. In other embodiments, the implantable medical device 1 may be a monitoring device, such as a so-called biomonitor for monitoring a physical function of the patient P, in particular a cardiac function. In yet other embodiments the implantable medical device 1 may be a neuro-stimulation device.

The external device 2 may for example be a wearable device, such as a smart watch device or a device to be worn on the chest of the patient P. In other embodiments, the external device 2 may be a smart phone or a tablet computer which may be carried by the patient.

Referring now to Fig. 2, the implantable medical device 1 comprises an implant processing circuitry 10, a first implant transceiver circuitry 11 and a second implant transceiver circuitry 12. The external device 2, in turn, comprises a device processing circuitry 20, a first device transceiver circuitry 21 and a second device transceiver circuitry 22.

The first implant transceiver circuitry 11 and the first device transceiver circuitry 21 generally are configured to establish a communication according to a first communication technique. For example, the first implant transceiver circuitry 11 and the first device transceiver circuitry 21 are configured for establishing a communication by an inductive coupling using inductive coil antennas in the first implant transceiver circuitry 11 and the first device transceiver circuitry 21. The first implant transceiver circuitry 11 and the first device transceiver circuitry 21 for example generally may be configured to establish a communication in the near field.

In contrast, the second implant transceiver circuitry 12 and the second device transceiver circuitry 22 may be configured to establish a communication according to a second communication technique, for example a wireless RF communication technique, such as according to MICS, BLE or a telemetry technique, and hence are generally configured to communicate over a wider spatial range.

In the system of Figs. 1 and 2, a secure communication for exchanging data in between the implantable medical device 1 and the external device 2 shall be established using the second implant transceiver circuitry 12 and the second device transceiver circuitry 22. For providing for an authentication of the communication using the second implant transceiver circuitry 12 and the second device transceiver circuitry 22, authentication tokens W2, W2' shall be generated, and based on the authentication tokens W2, W2' an authentication between the implantable medical device 1 and the external device 2 shall take place.

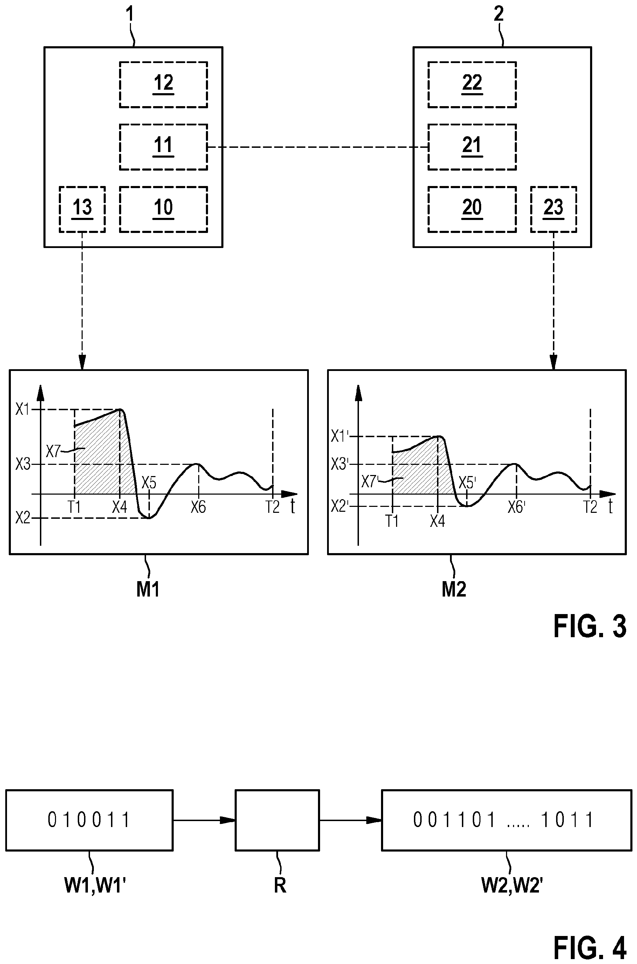

Referring now to Fig. 3, the implantable medical device 1 comprises an implant motion sensor 13, and the external device 2 comprises a device motion sensor 23, each motion sensor 13, 23 being configured to sense a motion signal M1, M2. As the implantable medical device 1, in an operative, implanted state, is implanted in the patient P, the motion signal M1 as sensed by the implant motion century 13 generally relates to the physical body motion of the patient P. Also, if the external device 2 is carried on the patient P, for example at a defined position on the chest of the patient P, the motion signal M2 as sensed by the device motion sensor 23 will relate to the physical body motion of the patient P. If the implantable medical device 1 and the external device 2 are in proximity to one another, for example if the implantable medical device 1 is a cardiac device implanted in or near the patient's heart and the external device 2 is placed on the chest of the patient P, it can be assumed that the motion signals M1, M2 as picked up by the motion sensors 13, 23 will be closely correlated with one another.

This can be used to derive authentication tokens W2, W2' based on the motion signals M1, M2 as sensed by the implantable medical device 1 and the external device 2.

As illustrated in Fig. 3, the implantable medical device 1, using the implant motion sensor 13, senses a motion signal M1 in a defined time range between time points T1 and T2. Likewise, the external device 2, using the device motion sensor 23, senses a motion signal M2 in the defined time range between time points T1 and T2. If the external device 2 is placed on the patient P in comparatively close proximity to the implantable medical device 1, the motion signals M1, M2 will be correlated with one another, because the motion sensors 13, 23 will generally sense a common body motion of the patient P.

In order to synchronize the time range for sensing the motion signals M1, M2, the implantable medical device 1 and the external device 2 are configured to establish a communication using the first implant transceiver circuitry 11 and the first device transceiver circuitry 21. By means of the first implant transceiver circuitry 11 and the first device transceiver circuitry 21, hence, information is exchanged in order to align the sensing of the motion signals M1, M2.

For example, the external device 2, using the first device transceiver circuitry 21, may transmit a trigger signal to the implantable medical device 1 indicating a starting point for the time range for measuring the motion signals M1, M2 and hence triggering the sensing of the motion signals M1, M2.

Based on the respective motion signal M1, M2, the implant processing circuitry 10 respectively the device processing circuitry 20 is configured to derive the authentication token W2, W2', for example by analyzing the respective motion signal M1, M2 to determine characteristic quantities X1...X7, X1'...X7', for example relating to maxima and minima in the motion signal M1, M2, to times of occurrence of the maxima and minima, to areas under certain portions of the curve of the motion signal M1, M2, or to the relative change in between maxima and/or minima, or the like.

Based on the characteristic quantities X1...X7, X1'...X7', authentication tokens W2, W2' may directly be derived. In other embodiments, based on the characteristic quantities X1...X7, X1'...X7' characteristic values may be determined, which may be used as a starting point for generating the authentication tokens W2, W2'.

Referring now to Fig. 4, in one embodiment the implant processing circuitry 10 and the device processing circuitry 20 each implement a randomizing function R, into which a characteristic value W1, W1' is fed as an input for generating an authentication token W2, W2'. The characteristic value W1, W1' may be determined according to characteristic quantities X1...X7, X1'...X7' derived from the motion signal M1, M2 and may be a comparatively small number, for example a number in a digital range between 1 to 1024, 1 to 256, 1 to 64, or 1 to 16, corresponding to a number of bits equal to 10, 8, 6 or 4. By means of the randomizing function R the characteristic value W1, W1' is randomized and a corresponding authentication token W2, W2' is determined, which is a substantially larger number, for example a number expressed by 32 bit, 64 bit or 128 bit.

The randomizing function R may be provided with further randomizing input information, for example information relating to the time of day or other information, such as patient information, to increase a degree of randomization.

By means of the authentication tokens W2, W2' an authenticated communication is established using the second implant transceiver circuitry 12 and the second device transceiver circuitry 22, for example using a wireless RF communication technique, such as a MICS scheme, BLE, or telemetry. Herein, for example using a private key/public key encryption technology the authentication tokens W2, W2' may be exchanged in between the implantable medical device 1 and the external device 2, and authentication is carried out based on a comparison of the authentication tokens W2, W2'. If the authentication is successful, i.e., if the authentication tokens W2, W2' match with one another, an authenticated communication between the implantable medical device 1 and the external device 2 using the second implant transceiver circuitry 12 and the second device transceiver circuitry 22 is established, for example for exchanging data such as programming data or operational data obtained at the implantable medical device 1 during operation.

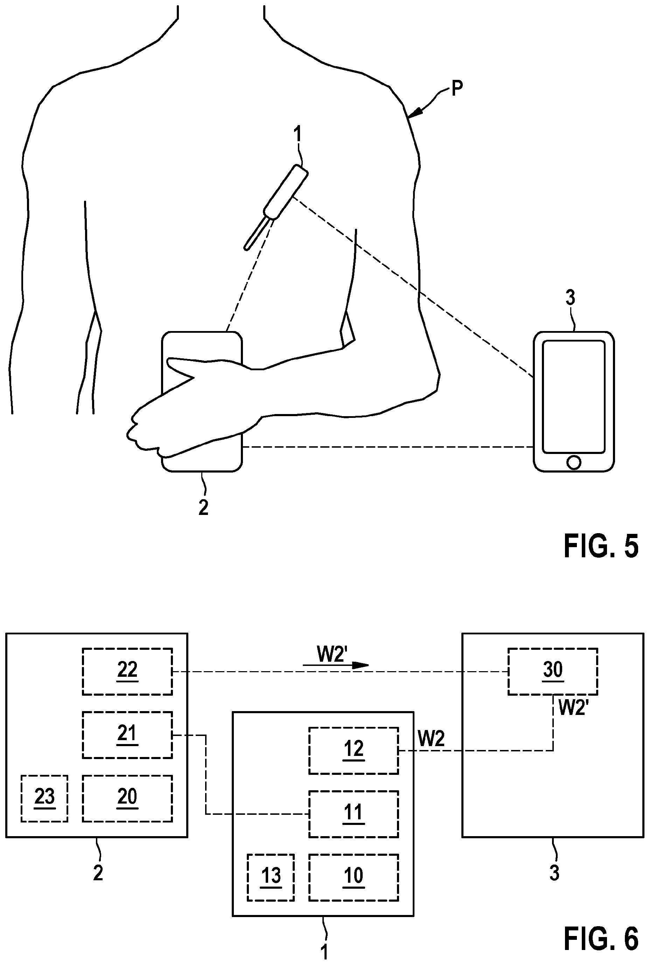

Referring now to Fig. 5, in a different setup a system may comprise an implantable medical device 1, an external device 2 and a further device 3. The implantable medical device 1 is configured for implantation in the patient P. The external device 2 is configured to operate externally to the patient P, but may be placed on the patient P, for example on the chest of the patient P, or may be a wearable device to be worn by the patient P at a defined location. The further device 3 may be communication device, such as a clinician programming device or a computer.

In the system according to Fig. 5, authentication tokens W2, W2' are generated at the implantable medical device 1 and the external device 2, just as it has been described before according to Figs. 1 to 4. However, rather than establishing an authenticated communication directly between the implantable medical device 1 and the external device 2, in the system of Fig. 5 the external device 2 transmits information relating to the authentication token W2' generated at the external device 2 to the further device 3, such that the further device 3 is enabled to establish an authenticated communication with the implantable medical device 1 based on the authentication tokens W2, W2'.

Referring now to Fig. 6, the authentication tokens W2, W2' are generated at the implantable medical device 1 and the external device 2, as it has been described before according to Figs. 1 to 4. Upon generating the authentication token W2', the external device 2 transmits information relating to the authentication token W2' to the further device 3, which, via a transceiver circuitry 30, establishes an authenticated communication with the second implant transceiver circuitry 12 of the implantable medical device 1.

In particular, for example using a private key/public key encryption technology the authentication tokens W2, W2' may be exchanged in between the second implant transceiver circuitry 12 and the transceiver circuitry 30 of the further device 3, such that an authentication may take place. If the authentication is successful, an authenticated communication is enabled, such that a data exchange between the further device 3 and the implantable medical device 1 may take place.

The further device 3 may for example comprise a user interface, such that a user, for example the patient P, may trigger the establishing of a communication. Accordingly, the further device 3 communicates with the external device 2 and prompts the external device 2 to start a process for generating authentication tokens W2, W2' in concert with the implantable medical device 1. Upon generating the authentication token W2', the external device 2 transmits information relating to the authentication token W2', e.g. the authentication token W2' itself, to the further device 3, for example using a secure communication connection via BLE or NFC, such that the further device 3 is enabled to establish a communication with the implantable medical device 1.

Because in any of the systems of Figs. 1 or 5 the external device 2, for generating the authentication tokens W2, W2', is required to be in the patient's possession, it can be assumed that the authentication tokens W2, W2' reliably may be used for providing an authentication for establishing a communication session.

### List of reference numerals

- 1: Implantable medical device
- 10: Implant processing circuitry
- 11: Implant transceiver circuitry
- 12: Implant transceiver circuitry
- 13: Implant motion sensor
- 2: External device
- 20: Device processing circuitry
- 21: Device transceiver circuitry
- 22: Device transceiver circuitry
- 23: Device motion sensor
- 3: Further device
- 30: Further transceiver circuitry
- M1, M2: Motion signal
- P: Patient
- R: Key generator
- t: Time
- T1, T2: Time point
- W1, W1': Input word
- W2, W2': Authentication token
- X1... X7, X1'... X7': Characteristic quantities

## Claims

1. A system for providing a therapeutic and/or diagnostic function on a patient (P), the system comprising:
an implantable medical device (1) configured for implantation in the patient (P) and having a first implant transceiver circuitry (11), a second implant transceiver circuitry (12), an implant processing circuitry (10) and an implant motion sensor (13);
an external device (2) having a first device transceiver circuitry (21), a device processing circuitry (20) and a device motion sensor (23);
wherein the implant processing circuitry (10) is configured to obtain, using the implant motion sensor (13) and based on a communication established via the first implant transceiver circuitry (11) and the first device transceiver circuitry (21), a first motion signal (M1) and to derive, based on the first motion signal (M1), a first authentication token (W2);
wherein the device processing circuitry (20) is configured to obtain, using the device motion sensor (23) and based on said communication established via the first implant transceiver circuitry (11) and the first device transceiver circuitry (21), a second motion signal (M2) and to derive, based on the second motion signal (M2), a second authentication token (W2');
wherein the implant processing circuitry (10) is configured to authenticate a communication via the second implant transceiver circuitry (12) based on the first authentication token (W2) and the second authentication token (W2').

2. The system according to claim 1, wherein first implant transceiver circuitry (11) and the first device transceiver circuitry (21) are configured to establish a communication employing an inductive coupling technique.

3. The system according to claim 1 or 2, wherein the device processing circuitry (20) is configured, using said communication established via the first implant transceiver circuitry (11) and the first device transceiver circuitry (21), to transmit information to the implant processing circuitry (10) relating to a time range for obtaining the first motion signal (M1) and the second motion signal (M2).

4. The system according to claim 3, wherein the device processing circuitry (20) is configured, using said communication established via the first implant transceiver circuitry (11) and the first device transceiver circuitry (21), to transmit a trigger signal to the implant processing circuitry (10) to start said time range for obtaining the first motion signal (M1) and the second motion signal (M2).

5. The system according to one of the preceding claims, wherein the second implant transceiver circuitry (12) is configured to establish a communication using a wireless RF communication technique.

6. The system according to one of the preceding claims, wherein the external device (2) comprises a second device transceiver circuitry (22).

7. The system according to claim 6, wherein the second implant transceiver circuitry (12) and the second device transceiver circuitry (22) are configured to establish an authenticated communication based on the first authentication token (W2) and the second authentication token (W2').

8. The system according to one of the preceding claims, wherein the external device (2) is configured to transmit information relating to the second authentication token (W2') to a further device (3).

9. The system according to claim 8, wherein the further device (3) comprises a further transceiver circuitry (30), wherein the second implant transceiver circuitry (12) and the further transceiver circuitry (30) are configured to establish an authenticated communication based on the first authentication token (W2) and the second authentication token (W2').

10. The system according to one of the preceding claims, wherein the implant processing circuitry (10) is configured to determine a first characteristic value (W1) based on the first motion signal (M1) and to derive said first authentication token (W2) based on the first characteristic value (W1), and the device processing circuitry (20) is configured to determine a second characteristic value (W1') based on the second motion signal (M2) and to derive said second authentication token (W2') based on the second characteristic value (W1').

11. The system according to claim 10, wherein the implant processing circuitry (10) is configured to determine said first characteristic value (W1) based on a set of first characteristic quantities (X1... X7) derived from the first motion signal (M1), and the device processing circuitry (20) is configured to determine said second characteristic value (W1') based on a set of second characteristic quantities (XI'... Xl') derived from the second motion signal (M2).

12. The system according to claim 10 or 11, wherein the implant processing circuitry (10) is configured to derive said first authentication token (W2) from the first characteristic value (W1) using a pre-defined randomizing function (R), and the device processing circuitry (20) is configured to derive said second authentication token (W2') from the second characteristic value (W1') using the same pre-defined randomizing function (R).

13. The system according to one of the preceding claims, wherein the external device (2) is a wearable device configured to be worn by the patient.

14. A method for operating a system for providing a therapeutic and/or diagnostic function on a patient (P), the method comprising:
providing an implantable medical device (1) configured for implantation in the patient (P) and having a first implant transceiver circuitry (11), a second implant transceiver circuitry (12), an implant processing circuitry (10) and an implant motion sensor (13);
providing an external device (2) having a first device transceiver circuitry (21), a device processing circuitry (20) and a device motion sensor (23);
obtaining, by the implant processing circuitry (10), a first motion signal (M1) using the implant motion sensor (13) and based on a communication established via the first implant transceiver circuitry (11) and the device transceiver circuitry (21), and deriving, based on the first motion signal (M1), a first authentication token (W2);
obtaining, by the device processing circuitry (20), a second motion signal (M2) using the device motion sensor (23) and based on said communication established via the first implant transceiver circuitry (11) and the first device transceiver circuitry (21), and deriving, based on the second motion signal (M2), a second authentication token (W2');
authenticating, by the implant processing circuitry (10), a communication via the second implant transceiver circuitry (12) based on the first authentication token (W2) and the second authentication token (W2').

15. The method according to claim 14, wherein the patient is caused to perform a defined motion in a specified time range for allowing the implant processing circuitry (10) to obtain the first motion signal (M1) and the device processing circuitry (20) to obtain the second motion signal (M2).
